# EUROPEAN PATENT SPECIFICATION

(11) **EP 0 380 932 B1**
(45) Date of publication and mention of the grant of the patent: **21.08.1996**
(21) Application number: 90100409.3
(22) Date of filing: 10.01.1990
(51) Int. Cl.: C12Q 1/04, C12Q 1/37

(54) **Solid phase test for the detection of periodontal disease in subgingival plaque**
Festephasentest zum Nachweis der periodentalen Krankheit in einem subgingivalem Fleck
Essai en phase solide pour la détection de maladies périodontales dans la plaque subgingivale

(30) Priority: 30.01.1989 US 303145
(43) Date of publication of application: 08.08.1990
(73) Proprietor: Loesche, Walter J., Ann Arbor, Michigan 48104 (US)
(72) Inventor: Kerschensteiner, Daniel, Wayne, PA 19087 (US)
(74) Representative: Denmark, James

(56) References cited:
- EP-A- 0 330 358
- DE-A- 1 923 104
- JOURNAL OF PERIODONTOLOGY, vol. 58, no. 4, April 1987; W.J. LOESCHE et al., pp. 266-273
- JOURNAL OF DENTAL RESEARCH, vol. 66, no. 11, 1987; W.A. BRETZ et al., pp. 1668-1672

## Description

### BACKGROUND OF THE INVENTION

This invention relates to an improved solid-phase assay for determining the presence of periodontal disease-causing organisms in subgingival plaque.

### DESCRIPTION OF THE PRIOR ART

Probing depth, attachment loss, and particularly bleeding upon probing have traditionally been used as indicators of periodontal diseases. However,these clinical parameters are more a reflection of past disease experience and the inflammatory status of the tissues, rather than providing information about the bacterial composition of the subgingival plaque.

Conventional microbiological procedures are effective in identifying periodontal pathogens in the plaque, but they suffer from the disadvantages of being time-consuming and requiring specialized personnel for their execution. Dark field microscopy allows for organisms with a characteristic morphology or motility to be viewed, counted and correlated with clinical parameters. However, the clinician often lacks the time to make a microscopic examination cost effective. Therefore, a quick and simple bacteriological test that could be reliably used in everyday practice to identify disease-indicator organisms would be highly desirable.

German Patent DE 1923104 discloses a semisolid phase test for detecting the presence of β-D-galactosidase in plaque by the hydrolysis of one of its substrates 6-halogen-2-naphthyl-β-D-Galactopyranoside which produces a chromaphore 6-halogen-2-naphthol which in turn reacts with an indicator after a two hour incubation perod in a water bath at temperature of 37°C.

Bacteriological studies show that spirochetes, Bacteroides gingivalis and Bacteroides forsythus are present in increased proportions in patients with clinical signs of periodontal disease [Pickett et al., J. Dent. Res., 65, 195 (1986); Dzink et al., J.Clin.Periodontol., 12 (1986), 648-659 (1985) and Slots et al., J. Clin. Periodontol., 12, 540-552 (1985)] and that these microorganisms are reduced following successful treatment and maintenance [Loesche et al., J. Periodontol., 55, 325-335 (1984)]. Therefore, a test confirming or eliminating their presence could help the clinician to monitor the adequacy of treatment procedures, as a negative test would indicate that those microorganisms have been significantly suppressed or eliminated from the subgingival plaque.

Three of the most important bacteria associated with periodontitis, Bacteroides gingivalis, Treponema denticola (a spirochete) and Bacteroides forsythus share the unique ability of hydrolyzing the trypsin substrate N-benzoyl-DL-arginine-2-naphthylamide (BANA).

Studies by Loesche et al., J.Periodontol., 58, 266-273 (1987) have demonstrated that the hydrolysis of the trypsin substrate N-benzoyl-DL-arginine-2-naphthylamide (BANA) by subgingival plaque obtained from a single site correlates best with the numbers and proportions of spirochetes in plaque samples and may serve as an indicator of clinical disease. Bretz and Loesche, J.Dent. Res., 66(11), 1668-1672 (1987) furthered such studies utilizing pooled subgingival plaque samples. Their method of detecting disease involved utilizing a liquid-phase assay which incubates the sample overnight with a BANA solution at pH 7.0 and detects the hydrolysis by the addition of a fast garnet color developer. Overnight incubation of this assay was found to be necessary for the indicative color change in 85% of their samples.

### SUMMARY OF THE INVENTION

In accordance with the present invention, a solid-phase assay for the detection of N-benzoyl-DL-arginine-2-naphthylamide (BANA) hydrolysis is prepared which is useful for detecting the presence of periodontal disease-causing organisms.

The assay comprises:
a) a first element incorporating a pH 8-9- buffered solution of N-benzoyl-DL-arginine-2-naphthylamide (BANA) for receiving samples of subgingival plaque;
b) a second element comprising a nylon or nitrocellulose membrane comprising an adsorbed colour developer which has an optimum pH of 6-9 for a coupling reaction with β-naphthylamine; whereby placement of a subgingival plaque sample on the first element followed by intimate contact with the wetted second element and incubation of the combined elements at 50-60°C for an incubation period of 5-30 minutes results in a color change on the second element, thus indicating that BANA hydrolysis has occurred.

The present invention also includes a solid phase assay method for the detection of BANA hydrolysis to detect the presence of periodontal disease causing organisms characterised by the steps of:
a) collecting a sample or samples of subgingival plaque from a patient;
b) applying said sample or samples to the first element which incorporates a pH 8-9-buffered solution of N-benzoyl-DL-arginine-2-naphthylamide (BANA);
c) providing a second element of the assay which comprises a nylon or nitrocellulose membrane comprising an adsorbed colour developer which has an optimum pH of 6-9 for a coupling reaction with β-naphthylamine;
d) intimately contacting said first element with said second element; and
e) incubating the combined first and second elements at 50-60°C for an incubation period of 5-30 minutes;
f) observing said second element to detect a color change indicating that BANA hydrolysis has occurred.

The invention further provides a kit for use in a solid phase assay for the detection of N-benzoyl-DL-arginine-2-naphthylamide (BANA) comprising:
a first element onto which a pH 8-9 buffered solution of BANA has been adsorbed; and
a second element comprising a nylon or nitrocellulose membrane onto which a solution of a colour developer which has an optimum pH of 6-9 for a coupling reaction with β-naphthylamine has been adsorbed.

Accordingly, it is a principal object of the present invention to provide a solid-phase assay for the detection of BANA hydrolysis.

It is a further object of the invention to provide a method of utilizing the solid-phase assay to detect and monitor the presence of periodontal disease-causing organisms.

It is a still further object of the invention to provide a solid-phase assay for the detection and monitoring of periodontal disease which can be performed in a relatively short period of time such as in a typical dental office visit.

It is another object of the invention to provide a solid-phase assay for the detection and monitoring of periodontal disease which is easily and visually read.

It is still another object of the invention to provide a solid-phase assay for the detection and monitoring of periodontal disease which provides a permanent stable record which is easily maintainable in a patient's files.

It is still another object of the invention to provide a solid-phase assay for the detection and monitoring of periodontal disease which can be performed easily and quickly by dental personnel during routine dental office visits.

These and other objects and advantages of the present invention will become more apparent from the following more detailed description thereof.

### DETAILED DESCRIPTION OF THE INVENTION

In its broad aspect, the present invention is directed to a solid-phase assay for the detection of N-benzoyl-DL-arginine-2-naphthylamide (BANA) hydrolysis, the process for its preparation and methods of use thereof.

The solid-phase assay comprises a first element having adsorbed thereto a pH 8-9-buffered solution of N-benzoyl-DL-arginine-2-naphthylamide (BANA) which receives the samples of a patient's subgingival plaque and a second element which is a nylon or nitrocellulose membrane containing adsorbed Fast Black K salt. When a sample of subgingival plaque containing a microorganism capable of hydrolyzing BANA is placed on the first element, hydrolysis occurs. When the second element, activated by water, contacts the first element the reaction product of the hydrolysis, β-naphthylamine, migrates onto the nylon or nitrocellulose membrane second element and produces a color change in the Fast Black K salt which is readily observed.

The invention also relates to a process for the preparation of the solid-phase assay which is accomplished by adsorbing a pH 8-9-buffered solution of BANA onto the first element and a Fast Black K salt solution onto the nylon or nitrocellulose membrane second element. Both elements are dried. Typically, they are packaged in such a manner so as to prevent light, air, water and other contaminants from reaching their surfaces until their use in the method of the present invention.

Additionally, the present invention also relates to a method of utilizing a BANA-hydrolysis detection solid-phase assay to detect the presence of periodontal disease-causing organisms.

The method involves collecting a sample or samples of subgingival plaque from a patient and applying said sample or samples to the first element of the assay which element incorporates a pH 8-9-buffered solution of N-benzoyl-DL-arginine-2-naphthylamide (BANA). Then, a second element of the assay which is a nylon or nitrocellulose membrane containing adsorbed Fast Black K salt is wetted and intimately contacted with the first element. The combined elements are then incubated at 50-60°C for an incubation period of about 5-30 minutes. When the incubation period is completed, the combined first and second elements are separated, and the second element is observed to detect a color change. A color change on the second element indicates that BANA hydrolysis has occurred. This indicates the presence of a periodontal disease-causing organism capable of hydrolyzing BANA.

The solid-phase assay of the present invention is able to confirm periodontal tissue health statues before, during and following treatment. Thus, the dentist and patient will be able to assess the periodontal tissue condition quickly and objectively. If test results remain positive following a course of treatment, then the treatment plan can be modified to minimize additional tissue destruction.

Additionally, the solid-phase assay of this invention possesses the ability to detect sites which are, in fact, infected with the disease-implicated microorganisms, but which do not, as yet, demonstrate clinical signs of the disease. In this way, the assay can provide early warning to the dentist, hygienist and patient that increased oral hygiene and/or prophylaxis are required to prevent or minimize the possible onset of clinical periodontal disease.

A potential use of the instant assay is to aid dentists and hygienists in patient education. Patients often have difficulty understanding the causes of periodontal disease and its treatment, especially when they experience no discomfort or other symptoms indicative of disease. When a means of visualizing the presence and extent of their disease on a site specific basis is available, it can serve to modify their behavior towards oral hygiene and professional dental care.

The first element of the solid-phase assay of the present invention is comprised of any water-adsorbable matrix material. Typically, such material is comprised of a cellulosic or polyester paper material. Paper, and particularly paper pads, have the advantages of being commercially available in a variety of suitable weights and absorbencies, many of which are FDA approved for diagnostic usage. They are also relatively cheap materials which function to absorb and stabilize upon drying the watersoluble enzyme-sensitive reagents which are utilized in the solid-phase assay of this invention. Suitable paper materials are those cellulosic papers available from Whatman Co. and Schleicher and Schuell Corp., and polyester papers such as those available from E & D. Corp. Particularly preferred cellulosic papers are those designated as S & S #903 and Whatman 3MM. Of course, other equivalent brands of paper material can be substituted in the practice of the present invention without affecting the operability of the solid-phase assay.

The N-benzoyl-DL-arginine-2-naphthylamide (BANA) utilized in the invention is a commercially available substrate supplied by companies such as Sigma Chemical Co. (St. Louis, MO). This substrate is hydrolyzed by its corresponding enzyme to produce β-naphthylamine. This hydrolysis is critical to the conduct of this assay. It optimally occurs with a pH of about 8-9. For this reason, the solid-phase assay of the present invention utilizes a Tris buffer of pH 8-9 in the practice of its invention. Tris buffer is a highly preferred buffering material due to its suitability for biological reaction and its general availability. Of course, other equivalent buffering materials or agents, e.g., sodium and potassium phosphates, borates and carbonates, which provide the necessary 8-9 pH can be used. Preferably the pH is maintained in the range of 8.5 to 8.8. The BANA is preferably applied to the first element so as to provide a concentration of about 1076-2152 mg/m² (100-200 mg/ft²) or about 30-60 mg/assay.

The second element of the present invention comprises a nylon or nitrocellulose membrane. This nylon or nitrocellulose membrane supports the Fast Black K salt which is the color developer for the solid-phase assay of the present invention.

Suitable nitrocellulose membranes are those available from Schleicher & Schuell Corp. and designated as BA95, 0.45 µm. Suitable nylon membranes are those sold by Schleicher & Schuell as "nylon 66" or Pall nylon 66 (Biodyne A^{TM}). The nylon or nitrocellulose membrane is negatively charged. These materials are thus highly acidic and favor the coupling of the Fast Black K salt color developer to the β-naphthylamine which is the product of the hydrolysis of the BANA. Additionally, these nylon and nitrocellulose membranes are capable of adsorbing a large amount of the Fast Black K salt color developer. This is critical to the sensitivity of the solid-phase assay. The nylon or nitrocellulose membranes also adsorb the Fast Black K salt along with its stabilizers which are included in the commercial product. This ability to adsorb the stabilizers such as zinc chloride (ZnCl₂) of the Fast Black K salt color developer as well as the Fast Black K salt color developer results in the finished second element retaining the stability of the initial reagent. This obviously contributes to the shelf life of the finished solid-phase assay.

The Fast Black K salt color developer utilized in the present invention as a component of the second element is a commercially available product available from such chemical suppliers as Sigma Chemical Co. (St. Louis, MO). It is of the category of an azodiazo dye color developer. The pH optimum of the coupling reaction of the diazonium salt and the arylamine is about 6. This pH optimum greatly contributes to the operability and stability of the solid-phase assay of the present invention. Since the hydrolysis of the present invention occurs at an optimum pH of about 8-9, it is critical that there not be a wide spread between the pH optimum of the color developer being utilized and the hydrolysis reaction. If a color developer with a lower pH optimum were utilized, there would be a physical deterioration of the color developer at the pH at which the hydrolysis occurs. This would obviously contribute to false readings in the resultant solid phase assay. Other equivalent color developers such as Fast Dark Blue R can be substituted for the Fast Black K salt color developer in the practice of the present invention, but they must meet the pH optimum requirements. That is, they must have a pH optimum of between about 6-9. The Fast Black K salt is typically utilized in the solid-phase assay of this invention in a concentration of about 215.2-322.8 mg/m²(20-30 mg/ft²) or 6-9 mg/assay.

An optional addition into the practice of the preparation of the solid-phase assay of the present invention is the addition of stabilizing agents to the Fast Black K salt solution being adsorbed by the nylon or nitrocellulose membrane. Typically, a polymer stabilizing agent is utilized. These are commercially available polyethylene glycols having a molecular weight of between 6000-8000 and such polyethylene acids as sold under the trademark Gantrez® for instance, Gantrez® S-95 (available from GAF Corp.). These polymer stabilizing agents are added to the solution of Fast Black K salt in an amount about 0.05-1.0% by weight. The polymer stabilizing agent acts to adsorb any water of decomposition and stabilize the second element (the nylon or nitrocellulose membrane) during storage before use. The polymer stabilizing agent also serves further to act as a wetting agent for smooth activation of the nylon or nitrocellulose membrane-Fast Black K salt color developer element immediately prior to its use in the conduct of the assay of the present invention.

A further optional additive to the second element of the assay of this invention is a diazo-dye stabilizer. Such diazo-dye stabilizers are commonly added to the commercially available solutions of dye color developers of the diazo type. Fast Black K salt is a diazo-type dye. Typical diazo-dye stabilizers are those such as 1,5-naphthylenedisulfonic acid and its salts as well as zinc chloride and other transition metal chlorides. These dye stabilizers can be present in the Fast Black K salt as purchased from the manufacturer or can be added during the preparation of the Fast Black K salt-nylon or nitrocellulose second element. Typically, such dye stabilizers are added in an amount which is about 0.1 to 1.0% by weight.

A further optional embodiment of the present invention is the use of sample retaining means on the first element. Such sample retaining means can be of a multitude of types. A particularly suitable type of sample retaining means, especially where the first element is comprised of a paper pad, is a sample retaining means in the form of an embossed dimple. Such dimples are typically embossed by manufacturing apparatus which impress the dimple into the paper pad prior to its adsorption of the Tris-buffered BANA. Such dimples embossed into the paper pad reduce the reaction volume into a minimum and ease the application of the subgingival plaque sample from a dental instrument onto the solid-phase assay.

The color produced by the coupling of the Fast Black K salt with the β-naphthylamine is blue-black on a salmon-colored background. This combination is easy to distinguish, particularly when samples are small in size and produce tests resulting in pinpricks of color.

Typically, in the conduct of preparing the solid-phase assay of the present invention, the individual elements will be prepared and then adhesively affixed to a plastic or other sturdy backing. Typically, this backing is a polystyrene card and the elements are affixed using double-sided adhesive tape. The elements are affixed, one on each side of perforations in the backing so that when the backing is folded along the perforations, the BANA first element is situated directly over the Fast Black K salt second element. This accomplishes two things: both elements of the assay are included together in any packaging, so that there is no chance of a dental practitioner utilizing two elements of the same type; and typically, the plastic backing contains perforations so that the second element can be exactly "folded over" at this hinge point to perform the step of intimately contacting the two elements required in the conduct of the present assay.

Such a plastic or other sturdy backing also facilitates the incorporation of the final assay into a patient's dental records. The elements are conveniently sized so as to provide a "sampling" card (to hold about 3-5 samples) or a "whole-mouth test" card (to hold about 30-32 samples).

The hydrolysis of the solid-phase assay of the present invention occurs most readily at a temperature of about 50-60° centrigrade. Thus, a source of heat of this temperature range is necessary for the conduct of the assay. Typically, this is provided by a dry heat incubator. Alternatively, a hot tap water bath (having a temperature of about 50-60°) can be utilized for the conduct of the assay. The use of this temperature reduces the time of completion and improves the sensitivity of the test since the hydrolysis occurs most quickly at this temperature and other by-products are reduced.

The following examples describe in detail the preparation of the solid-phase assay of the present invention and methods of use therefor.

### EXAMPLE 1

### Preparation of Solid-Phase Assay

### Materials:

Schleicher and Schuell Paper #903
Schleicher and Schuell Pure Nitrocellulose BA 95, 0.45 um
Fast Black K salt (Sigma)
BANA substrate (Sigma)
Tris base
1.0 M hydrochloric acid
Polyethylene glycol (6-8000 mw) [Sigma]
Medium weigh pans
Double adhesive stick tape (3M)
Hot water bath

### Method:

The paper and nitrocellulose membrane are cut into 5 x 5 cm squares.

### BANA Paper Preparation

A BANA solution is prepared by dissolving 44 mg of BANA in 1 ml of dimethylsulfoxide. This solution is then diluted 1:50 in 15 mM Tris-HCl, pH 8.5. One ml of the resulting solution is added to the bottom of a medium weigh pan, and into this is placed one square of paper #903. Absorption occurs in about 2 minutes time. The fluid-filled paper is then placed onto a glass surface, and the water is driven off at room temperature by dehydration using a dehumidifier.

Note: The paper can be used flat or a series of dimples can be molded into them using grommet pliers or other suitable molding apparatus. This is done before BANA impregnation by squeezing the grommet pliers partially through two layers of the paper to avoid breakthrough.

### Fast Black - Nitrocellulose Membrane Preparation

Fast Black is adsorbed onto the nylon or nitrocellulose squares by incorporating 9 squares of membrane to 20 ml of 10% polyethylene glycol, aqueous (6000-8000 molecular weight) containing 0.2% Fast Black K salt. This solution is clarified before use by passing it through a column fitted with a cotton plug to remove insoluble material.

The membranes are first prepared for adsorption by wetting in distilled water for one hour. Then the wet membranes and Fast Black K solution are combined in a medium weigh pan, covered with aluminum foil and placed in a 37°C water bath for 2-6 hours. After this the membranes are transferred, using forceps, to a sheet of filter paper and dried overnight at ambient room temperature.

The test is ready for use at this point, utilizing one each of the BANA paper and Fast Black K-nitrocellulose membrane.

### Sensitivity

Using this 30-minute test procedure to assess trypsin-like activity with known measured solutions of bovine pancrease trypsin (Type III, Sigma Chemical Co, approximately 11,680 BAEE units/mg protein), a detection limit of from 10 to 50 nanograms per 5 µl spot is determined. This is approximately a 100-fold improvement in the sensitivity of a liquid-based test performed at 37°C and 18-22 hours.

The ratio of trypsin hydrolysis of a BANA substrate compared to that of a purified Treponema denticola enzyme has been determined; see Purification and Characterization of an Enzyme Produced by Treponema denticola Capable of Hydrolyzing synthetic Trypsin Substrates." Ohta, K., Makinen, K.K., and Loesche, W.J., Infection and Immunity, 53, 213-220 (1986). This ratio of 11.7 means that the sensitivity of the test for this enzyme is 117-585 nanograms.

### EXAMPLE 2

### BANA Paper Preparation

Add the Tris buffer to distilled water and mix until dissolved. Titrate the solution to the pH range of 8.8 ± 0.2 with 2N HCl. Add the BANA (5.5 g/l) to methanol (90% w/v) with mixing until the BANA is dissolved. Slowly add the Tris buffer solution (0.1L) to the BANA methanol solution (0.9L). Mix thoroughly.

Using Whatman 3MM paper, the above solution is coated onto the paper at a rate of 1 liter/27.4 m (90 linear feet) of 15.24 cm (6 inch) wide paper and dried at a temperature of 82.2°C (180°F).

### Fast Black K Salt-Nylon Preparation

Gantrez® S-95 is added to methanol slowly and with mixing. Distilled water is then added with further stirring. To this solution is added 1,5-naphthylenediSO₃Na. After mixing, the Fast Black K salt is then added and stirred for 15-20 minutes. The resultant solution is covered tightly, protected from light, and used in the coating process as soon as possible.

Using Pall nylon membrane (Biodyne A^{tm}), the above solution is first filtered through a 40 micrometer filter and then coated onto the membrane at a rate of 0.0305-0.305 m/min (0.1-1 feet/minute), 1 litre/54.9 linear metres (1 liter/180 linear feet) of 15.24 cm (6 inch) membrane and dried at a temperature of 76.7°C (170°F). The impregnation is carried out under reduced lighting conditions (light intensities less than 21.52 lux (2 foot candles)). The dried impregnated nylon membrane is stored at ≤ 13% relative humidity. The coated paper and impregnated nylon membrane are cut into 1.27 cm (1/2") wide strips and laminated, one strip of each, adjacently on 11.43x13.34 cm (4.5" x 5.25") polystyrene cards.

### EXAMPLE 3

### Test Procedure:

Samples of subgingival plaque are collected and pooled from three or four sites per patient by means of a curette after the supragingival plaque has been removed and discarded. The plaque is collected in Sorensen phosphate buffer. Samples are applied to several portions of the BANA-containing first element. The Fast Black K-nylon or nitrocellulose membrane second element (activated by wetting with distilled water) is placed onto the sample-containing BANA first element. The combined elements are then placed in a dry heat incubator at 55°C for fifteen minutes. Alternatively, the combined elements can be placed in a hot tap water bath (about 55-59°C) for about thirty minutes. After the necessary length of time, the elements are removed from either the incubator or the bath, separated, and the Fast Black-nylon or nitrocellulose membrane second element is examined. The presence of dark blue spots on the Fast Black-nylon or nitrocellulose membrane second element indicates BANA hydrolysis and the presence of periodontal disease-causing organisms.

### EXAMPLE 4

### Solid Phase Assay Correlation with Clinical Diagnosis of Periodontal Disease

Ten plaque samples were obtained from laboratory personnel at Michigan (#1-10) and 15 plaques obtained from patients at Detroit (#11-25). The parameters measured include the clinician's judgment of health or disease, the pocket depth, bleeding upon probing, the number of spirochetes per high power microscopic field (hpf) (one spirochete/hpf is approximately 1x10⁶ organisms), the color intensity of the Fast Black K solid-phase assay, and the color intensity of the BANA reaction as conducted by overnight liquid-phase assay.

The plaque was collected in 110 µl of fluid, dispersed by vortexing and 10 µl was removed for microscopic counting of spirochetes only; another 50 µl was incubated overnight with the BANA in a liquid-phase assay. The remaining 50 µl was centrifuged and the resultant pellet is applied to the solid-phase assay and developed by the water method for 30 minutes. The results are given below in Table 1.

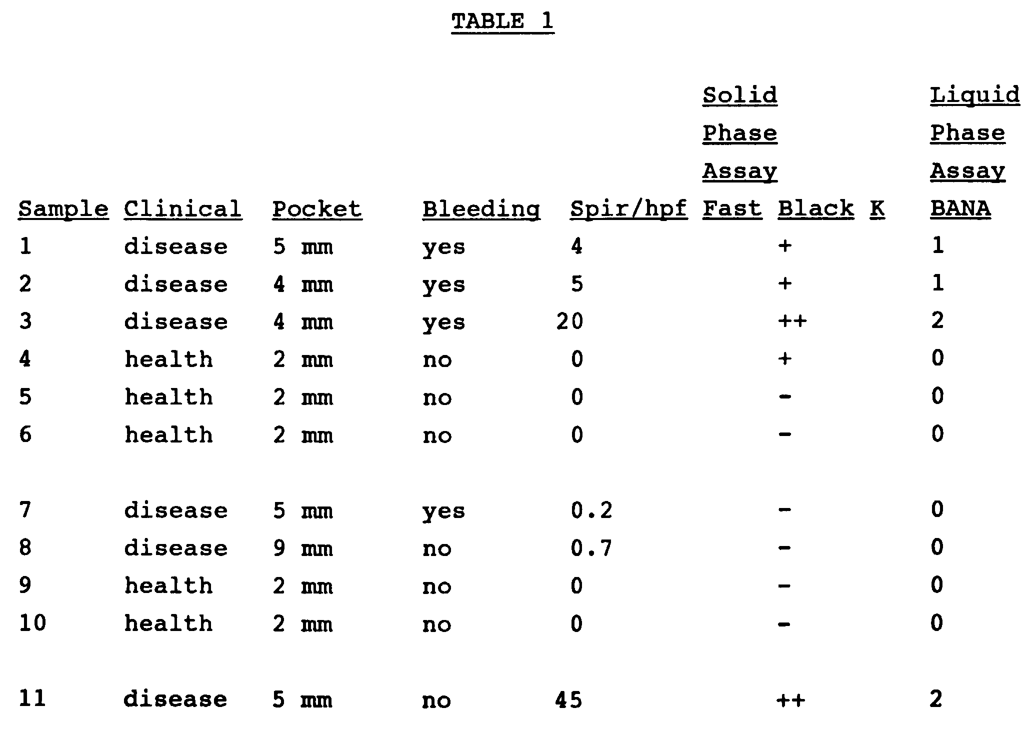

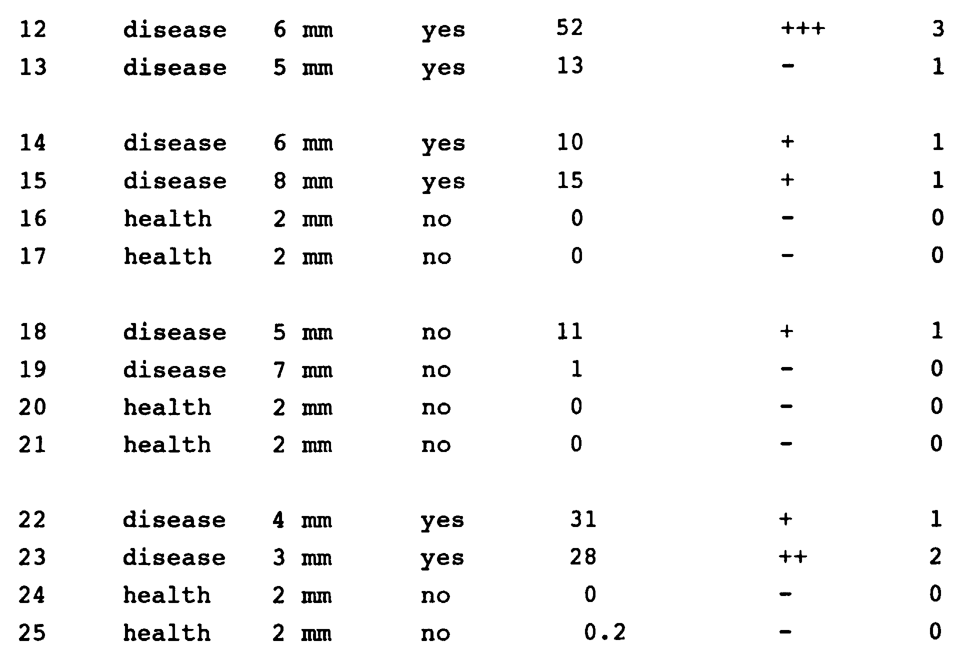

There are two specimens (plaques) in which the Fast Black K solid-phase assay differs from the liquid-phase assay and in which the Fast Black K solid-phase assay appears to be incorrect. In sample 4, the clinician judged the plaque to be from a healthy site, there were no detectable spirochetes, the liquid-phase assay was negative, but the Fast Black K solid-phase delay was a weak positive. This is a false positive. In sample 13, the clinician judged the site to be diseased, it bled upon probing, had high levels of spirochetes and was weakly positive by the liquid-phase assay, but negative by the Fast Black K solid-phase assay. This is a false negative.

Overall, there was agreement between the two tests and the majority of clinical parametres in 23 of 25 samples. A series of chi square analyses shows that there is a highly significant relationship between these tests and the various clinical parameters. There are three samples (#7, 8, 19) in which the Fast Black K solid-phase assay and liquid-phase assay were negative, but the clinical indications were mainly those of a disease site. In each instance the number of spirochetes/hpf was less than or equal to one, suggesting that the plaque sample was too small to give a reaction in the time used for incubation. This will always be a problem with very small plaque samples which can be minimized by multiple sampling techniques.

## Claims

1. A solid-phase assay for the detection of N-benzoyl-DL-arginine-2-naphthylamide (BANA) hydrolysis which comprises:
a first element incorporating a pH 8-9-buffered solution of N-benzoyl-DL-arginine-2-naphthylamide (BANA) for receiving samples of subgingival plaque; and
a second element comprising a nylon or nitrocellulose membrane comprising an adsorbed colour developer which has an optimum pH of 6 - 9 for a coupling reaction with β-naphthylamine;
whereby placement of a subgingival plaque sample on the first element followed by intimate contact with the wetted second element and incubation of the combined elements at 50-60°C for an incubation of 5-30 minutes results in a color change on the second element, thus indicating that BANA hydrolysis has occurred.

2. An assay in accordance with claim 1 wherein said colour developer is a diazonium salt.

3. An assay in accordance with claims 1 or 2 wherein said colour developer is Fast Black K salt or Fast Dark Blue R salt.

4. An assay accordance with Claims 1, 2 or 3 wherein the first element comprises a cellulosic paper pad.

5. An assay according to Claim 1, 2, 3 or 4 wherein the first element contains sample retaining means adapted to receive the samples of subgingival plaque.

6. An assay according to Claim 5 wherein the sample retaining means are dimples impressed into the first element.

7. An assay according to any of Claims 1-6, wherein the buffer is a Tris buffer.

8. An assay according to any of Claims 1-7 wherein the second element comprising a nylon or nitrocellulose membrane additionally contains a polymer stabilizing agent.

9. A solid phase assay method for the detection of BANA hydrolysis to detect the presence of periodontal disease causing organisms characterised by the steps of:
a) collecting a sample or samples of subgingival plaque from a patient;
b) applying said sample or samples to the first element which incorporates a pH 8-9-buffered solution of N-benzoyl-DL-arginine-2-naphthylamide (BANA);
c) providing a second element of the assay which comprises a nylon or nitrocellulose membrane comprising an adsorbed colour developer which has an optimum pH of 6-9 for a coupling reaction with β-naphthylamine;
d) intimately contacting said first element with said second element; and
e) incubating the combined first and second elements at 50-60°C for an incubation period of 5-30 minutes;
f) observing said second element to detect a color change indicating that BANA hydrolysis has occurred.

10. A method in accordance with claim 9 wherein said colour developer is a diazonium salt.

11. A method in accordance with claims 9 or 10 wherein said colour developer is Fast Black K salt or Fast Dark Blue R salt.

12. A method according to Claims 9-11, characterised in that the second element is wetted before said contacting step.

13. A method according to Claims 9-12 wherein the first element is a cellulosic paper pad.

14. A method according to Claims 9-13 wherein the first element contains sample retaining means adapted to receive the samples of subgingival plaque.

15. A method according to Claim 14 wherein the sample retaining means are dimples impressed into the first element.

16. A method according to Claims 9-15 wherein a Tris buffer is used to make up the solution of BANA.

17. A method according to any of Claims 9-16 wherein the second element additionally contains a polymer stabilizing agent.

18. A method according to any of Claims 9-17 wherein the incubation is conducted in a dry heat incubator.

19. A method according to any of claims 9 to 18 wherein the incubation is conducted in a hot tap water bath.

20. A process for the preparation of a solid-phase assay for the detection of N-benzoyl-DL-arginine-2-naphthylamide (BANA) hydrolysis which comprises:
adsorbing onto a first element a pH 8-9-buffered solution of N-benzoyl-DL-arginine-2-naphthylamide (BANA) and drying said element; and
adsorbing onto a second element comprising a nylon or nitrocellulose membrane a solution of a colour developer which has an optimum pH of 6-9 for a coupling reaction with naphthylamine; and drying said element.

21. A process in accordance with claim 20 wherein said colour developer is a diazonium salt.

22. A process in accordance with claims 20 or 21 wherein said colour developer is Fast Black K salt or Fast Dark Blue R salt.

23. A process according to Claims 20-22 wherein the first element is a cellulosic paper pad.

24. A process according to Claim 20-23 wherein the first element additionally contains sample retaining means.

25. A process according to Claim 24 wherein the sample retaining means are dimples embossed into the first element.

26. A process according to any of Claims 20-25 wherein a Tris buffer is used to make up the solution of BANA.

27. A process according to any of Claims 20-26 wherein the solution of colour developer additionally contains a polymer stabilizing agent.

28. A kit for use as a solid phase assay for the detection of the hydrolysis of N-benzoyl-DL-arginine-2-naphthylamide (BANA) comprising:
a first element onto which a pH 8-9-buffered solution of N-benzoyl-DL-arginine-2-naphthylamide (BANA) has been adsorbed; and
a second element comprising a nylon or nitrocellulose membrane onto which a solution of a colour developer which has an optimum pH of 6-9 for a coupling reaction with β-naphthylamine has been adsorbed.

29. A kit in accordance with claim 28 wherein said colour developer is a diazonium salt.

30. A kit in accordance with claims 28 or 29 wherein said colour developer is Fast Black K salt or Fast Dark Blue R salt.

31. A kit in accordance with claims 28-30 wherein each element is packaged in such a manner to prevent the ingress of light, air, water and/or other contaminents onto a surface thereof prior to their use in the method as claimed in claims 9-17.

32. A kit in accordance with claims 28-31 wherein the first element comprises a cellulosic or polyester paper material.

33. A kit according to Claim 28-32 wherein the first element contains sample retaining means adapted to receive the samples of subgingival plaque.

34. A kit according to Claim 33 wherein the sample retaining means are dimples impressed into the first element.

35. A kit according to any of Claims 28-34 wherein the second element comprising a nylon or nitrocellulose membrane additionally contains a polymer stabilizing agent.

36. A kit according to any of Claims 28-35 wherein each element is adhesively fixed to a plastic or the like sturdy backing material.

37. A kit according to Claim 36 wherein said backing material is polystyrene card.

38. A kit according to any of Claims 36 or 37 wherein the elements are symmetrically fixed to the backing material either side of a line of perforations such that when the backing material is folded along said line of perforations said first and second elements may be brought into direct contact.

39. A first element for use in a solid phase assay for the detection of the hydrolysis of N-benzoyl-DL-arginine-2-naphthylamide (BANA) when in combination with a second element which contains an adsorbed colour developer, wherein a solution of pH 6-9 buffered BANA is adsorbed in said first element.

## Patentansprüche

1. Feststoffprobe zum Nachweis einer Hydrolyse von N-Benzoyl-DL-Arginin-2-Naphtylamid (BANA), mit
- einem ersten Element, welches eine auf einen pH-Wert von 8 bis 9 gepufferte Lösung von N-Benzoyl-DL-Arginin-2-Naphtylamid (BANA) zur Aufnahme von Untersuchungsproben von Subgingivaplaques aufweist, und
- einem zweiten Element mit einer Nylon- oder Nitrozellulosemembrane, welches einen adsorbierten Farbentwickler aufweist, der einen optimalen pH-Wert von 6 bis 9 für eine Verbindungsreaktion mit β-Naphtylamin besitzt,
wobei eine Anbringung einer Subgingivaplaque-Probe auf dem ersten Element mit nachfolgendem innigen Kontakt mit dem angefeuchteten zweiten Element und eine Inkubation von 5 bis 30 Minuten zu einem Farbumschlag an dem zweiten Element führt, woraus ersichtlich ist, daß eine BANA-Hydrolyse stattgefunden hat.

2. Feststoffprobe nach Anspruch 1, wobei der Farbentwickler ein Diazonium-Salz ist.

3. Feststoffprobe nach Anspruch 1 oder 2, wobei der Farbentwickler ein lichtbeständiges schwarzes K-Salz oder ein lichtbeständiges dunkelblaues R-Salz ist.

4. Feststoffprobe nach Ansprüchen 1, 2 oder 3, wobei das erste Element ein Zellulosepapierkissen aufweist.

5. Feststoffprobe nach Ansprüchen 1, 2, 3 oder 4, wobei das erste Element eine Probenhalteeinrichtung aufweist, die zur Aufnahme der Untersuchungsproben von Subgingivaplaques dient.

6. Feststoffprobe nach Anspruch 5, wobei die Probenhalteneinrichtung aus Vertiefungen besteht, die in das erste Element eingeprägt sind.

7. Feststoffprobe nach einem der Ansprüche 1 bis 6, wobei als Puffer ein Tris-Puffer vorgesehen ist.

8. Feststoffprobe nach einem der Ansprüche 1 bis 7, wobei das zweite Element zusätzlich zu der Nylon- oder Nitrozellulose-Membrane einen polymeren Stabilisator aufweist.

9. Feststoff-Prüfverfahren für den Nachweis einer BANA-Hydrolyse zum Feststellen des Vorhandenseins von peridontalen Krankheitserregern, **gekennzeichnet durch** folgende Verfahrensschritte:
a) Von einem Patienten werden eine oder mehrere Untersuchungsproben von Subgingivaplaques gesammelt;
b) die Untersuchungsprobe(n) werden auf einem ersten Element einer Feststoffprobe angebracht, welches eine auf einen pH-Wert von 8 bis 9 gepufferte Lösung von N-Benzoyl-DL-Arginin-2-Naphtylamid (BANA) aufweist;
c) die Feststoffprobe wird mit einem zweiten Element versehen, welche eine Nylon- oder Nitrozellulose-Membrane mit einem adsorbierten Farbentwickler aufweist, der einen optimalen pH-Wert von 6 bis 9 für eine Verbindungsreaktion mit β-Naphtylamin besitzt;
d) das erste Element wird in innigen Kontakt mit dem zweiten Element gebracht, und
e) die miteinander vereinigten ersten und zweiten Elemente werden bei 50°C bis 60°C über eine Inkubationszeit von 5 - 30 Minuten bebrütet, wobei
f) das zweite Element beobachtet wird, um einen Farbumschlag festzustellen, welcher anzeigt, daß eine BANA-Hydrolyse stattgefunden hat.

10. Verfahren nach Anspruch 9, wobei als Farbentwickler ein Diazonium-Salz vorgesehen wird.

11. Verfahren nach Anspruch 9 oder 10, wobei als Farbentwickler ein lichtbeständiges schwarzes K-Salz oder ein lichtbeständiges dunkelblaues R-Salz vorgesehen wird.

12. Verfahren nach den Ansprüchen 9 bis 11, dadurch gekennzeichnet, daß das zweite Element vor dem Kontaktierungsschritt angefeuchtet wird.

13. Verfahren nach Ansprüchen 9 - 12, wobei als erstes Element ein Zellulosepapierkissen vorgesehen wird.

14. Verfahrne nach den Ansprüchen 9 bis 13, wobei das erste Element eine Probenhalteeinrichtung aufweist, die zur Aufnahme der Untersuchungsproben von Subgingivaplaques dient.

15. Verfahren nach Anspruch 14, wobei als Probenhalteeinrichtung Vertiefungen vorgesehen werden, die in das erste Element eingeprägt werden.

16. Verfahren nach den Ansprüchen 9 bis 15, wobei zur Herstellung der BANA-Lösung ein Tris-Puffer verwendet wird.

17. Verfahren nach einem der Ansprüche 9 bis 16, wobei das zweite Element zusätzlich mit einem polymeren Stabilisator versehen wird.

18. Verfahren nach einem der Ansprüche 9 bis 17, wobei die Inkubation in einem Trockenwärmeinkubator durchgeführt wird.

19. Verfahren nach einem der Ansprüche 9 bis 18, wobei die Inkubation in einem Wasserbad mit Warmwasserzapfhahn durchgeführt wird.

20. Verfahren zur Herstellung einer Feststoffprobe zum Nachweis einer Hydrolyse von N-Benzoyl-DL-Arginin-2-Naphtylamin (BANA), welches folgende Schnitte aufweist:
- Auf einem ersten Element wird eine auf einen pH-Wert von 8 bis 9 gepufferte Lösung von N-Benzoyl-DL-Arginin-2-Naphtylamin (BANA) adsorbiert und das Element getrocknet, und
- auf einem zweiten Element mit einer Nylon- oder Nitrozellulosemembrane wird eine Lösung eines Farbentwicklers adsorbiert, welche einen optimalen pH-Wert von 6 bis 9 für eine Verbindungsraktion mit β-Naphtylamin aufweist, wobei anschließend das Element getrocknet wird.

21. Herstellungsverfahren nach Anspruch 20, wobei als Farbentwickler ein Diazonium-Salz vorgesehen wird.

22. Herstellungsverfahren nach Anspruch 20 oder 21, wobei als Farbentwickler ein lichtbeständiges scharzes K-Salz oder ein lichtbeständiges dunkelblaues R-Salz vorgesehen wird.

23. Herstellungsverfahren nach den Ansprüchen 20 bis 22, wobei als erstes Element ein Zellulosepapierkissen vorgesehen wird.

24. Herstellungsverfahren nach den Ansprüchen 20 bis 23, wobei das erste Element zusätzlich mit einer Probenhalteeinrichtung versehen wird.

25. Herstellungsverfahren nach Anspruch 24, wobei als Probenhalteeinrichtung Vertiefungen vorgesehen werden, welche in das erste Element eingeprägt werden.

26. Herstellungsverfahren nach einem der Ansprüche 20 bis 25, wobei zur Herstellung der BANA-Lösung ein Tris-Puffer verwendet wird.

27. Herstellungsverfahren nach einem der Ansprüche 20 bis 26, wobei die Lösung des Farbentwicklers zusätzlich mit einem polymeren Stabilisator versehen wird.

28. Bausatz zur Verwendung als Feststoffprobe zum Nachweis der Hydrolyse von N-Benzoyl-DL-Arginin-2-Naphtylamin (BANA), mit
- einem ersten Element, auf dem eine auf einen pH-Wert von 8 bis 9 gepufferte Lösung von N-Benzoyl-DL-Arginin-2-Naphtylamin adsorbiert ist;
- einem zweiten Element mit einer Nylon- oder Nitrozellulosemembran, auf dem eine Lösung eines Farbentwicklers adsorbiert ist, welcher einen optimalen pH-Wert von 6 bis 9 für eine Verbindungsreaktion mit β-Naphtylamin aufweist.

29. Bausatz nach Anspruch 28, wobei der Farbentwickler ein Diazonium-Salz ist.

30. Bausatz nach Anspruch 28 oder 29, wobei der Farbentwickler ein lichtbeständiges schwarzes K-Salz oder ein lichtbeständiges dunkelblaues R-Salz ist.

31. Bausatz nach den Ansprüchen 28 bis 30, wobei jedes Element derart verpackt ist, daß der Zutritt von Licht, Luft, Wasser und/oder anderen Verunreinigungen auf eine Oberfläche der Elemente vor deren Benutzung bei dem Verfahren nach den Ansprüchen 9 bis 17 vermieden wird.

32. Bausatz nach den Ansprüchen 28 bis 31, wobei das erste Element ein Zellulose- oder Polyesterpapiermaterial aufweist.

33. Bausatz nach den Ansprüchen 28 bis 32, wobei das erste Element eine Probenhalteeinrichtung aufweist, die zur Aufnahme der Untersuchungsproben von Subgingivaplaques dient.

34. Bausatz nach einem der Ansprüche 28 bis 35, wobei die Probenhalteeinrichtung aus Vertiefungen besteht, die in das erste Element eingeprägt sind.

35. Bausatz nach einem der Ansprüche 28 bis 34, wobei das zweite Element zusätzlich zu der Nylon- oder Nitrozellulose-Membrane einen polymeren Stabilisator aufweist.

36. Bausatz nach einem der Ansprüche 28 bis 35, wobei jedes Element auf einem stabilen Trägermaterial aus Kunststoff oder dergleichen festgeklebt ist.

37. Bausatz nach Anspruch 36, wobei das Trägermaterial eine Polystyrolkarte ist.

38. Bausatz nach einem der Ansprüche 36 oder 37, wobei die Elemente symmetrisch auf dem Trägermaterial zu beiden Seiten einer Perforationslinie derart befestigt sind, daß beim Falten des Trägermaterials längs der Perforationslinie das erste und zweite Element in direkten Kontakt miteinander gebracht werden.

39. Verwendung eines ersten Elementes bei einer Feststoffprobe zum Nachweis der Hydrolyse von N-Benzoyl-DL-Arginin-2-Naphtylamin (BANA) in Kombination mit einem zweiten Element, das einen adsorbierten Farbentwickler enthält, wobei in dem ersten Element eine auf einen pH-Wert von 6 bis 9 gepufferte BANA-Lösung adsorbiert ist.

## Revendications

1. Analyse en phase solide destinée à la détection d'hydrolyse de N-benzoyl-DL-arginine-2-naphthylamide (BANA) qui comprend :
un premier élément incorporant une solution tamponnée avec pH de 8 à 9 de N-benzoyl-DL-arginine-2-naphthylamide (BANA) pour recevoir des échantillons de plaque sous-gingivale; et
un second élément comprenant une membrane en nylon ou en nitrocellulose comprenant un développeur de couleur absorbée qui a un pH optimal de 6-9 pour une réaction de couplage avec la naphthylamine;
de manière que le placement d'un échantillon de plaque sous-gingivale sur le premier élément suivi par un contact intime avec le second élément humide et une incubation de 5 à 30 minutes entraîne un changement de couleur sur le second élément, indiquant ainsi que l'hydrolyse BANA a eu lieu.

2. Analyse suivant la revendication 1, dans laquelle ledit développeur de couleur est un sel de diazonium.

3. Analyse suivant la revendication 1 ou 2, dans laquelle ledit développeur de couleur est le sel de Fast Black K ou le sel de Fast Dark Blue R.

4. Analyse suivant la revendication 1, 2 ou 3, dans laquelle le premier élément comprend un tampon de papier cellulosique.

5. Analyse suivant la revendication 1, 2, 3 ou 4, dans laquelle le premier élément contient des moyens de fixation d'échantillon adaptés pour recevoir les échantillons de plaque sous-gingivale.

6. Analyse suivant la revendication 5, dans laquelle les moyens de fixation d'échantillon sont des fossettes imprimées dans le premier élément.

7. Analyse suivant l'une quelconque des revendication 1 à 6, dans laquelle le tampon est un tampon Tris.

8. Analyse suivant l'une quelconque des revendication 1 à 7, dans laquelle le second élément comprenant une membrane de nylon ou de nitrocellulose contient, de plus, un agent stabilisant polymère.

9. Analyse en phase solide destinée à la détection d'hydrolyse de N-benzoyl-DL-arginine-2-naphthylamide (BANA) pour détecter la présence d'organismes causant une affection périodentale, caractérisée par les phases suivantes:
a) recueillir un échantillon ou des échantillons de plaque sous-gingivale du patient;
b) appliquer ledit échantillon ou lesdits échantillons à un premier élément qui incorpore une solution de N-benzoyl-DL-arginine-2-naphthylamide (BANA) tamponnée à un pH de 8 à 9;
c) prévoir un second élément de l'analyse qui comprend une membrane de nylon ou de nitrocellulose comprenant un développeur de couleur absorbée qui a un pH optimum de 6 à 9 pour une réaction de couplage avec la naphthylamine;
d) mettre en ccntact intime ledit premier élément avec ledit second élément; et
e) faire incuber les premier élément et second élément combinés à 50 à 60° C pendant un temps d'incubation de 5 à 39 minutes;
f) observer ledit second élément pour détecter un changement de couleur indiquant que l'hydrolyse BANA a eu lieu.

10. Méthode suivant la revendication 9, dans laquelle ledit développeur de couleur est un sel de diazonium.

11. Méthode suivant la revendication 9 ou 10, dans laquelle ledit développeur de couleur est un sel de Fast Black K ou un sel de Fast Dark Blue R.

12. Méthode suivant les revendications 9 à 11, caractérisée en ce que le second élément est humidifié avant ladite phase de mise en contact.

13. Méthode suivant les revendications 9 à 12, dans laquelle le premier élément est un tampon de papier cellulosique.

14. Méthode suivant les revendications 9 à 13, dans laquelle le premier élément contient des moyens de fixation d'échantillon adapte pour recevoir les échantillons de plaque sous-gingivale.

15. Méthode suivant la revendication 14, dans laquelle les moyens de fixation d'échantillon sont des fossettes imprimées dans le premier élément.

16. Méthode suivant les revendications 9 à 15, dans laquelle le tampon Tris est utilisé pour confectionner la solution de BANA.

17. Méthode suivant l'une quelconque de revendications 9 à 16, dans laquelle le second élément contient, de plus, un agent stabililisant polymère.

18. Méthode suivant l'une quelconque des revendications 9 à 17, dans laquelle l'incubation est réalisée dans un incubateur à chaleur sèche.

19. Méthode suivant l'une quelconque des revendications 9 à 18, dans laquelle l'incubation est réalisée dans un bain d'eau du robinet d'eau chaude.

20. Procédé pour la préparation d'une analyse en phase solide pour la détection d'hydrolyse N-benzoyl-DL-arginine-2-naphthylamide (BANA) qui consiste:
à absorber sur un premier élément une solution de N-benzoyl-DL-arginine-2-naphthylamide (BANA) tamponnée à un pH de 8 à 9 et à sécher ledit élément; et
à absorber sur un second élément comprenant une membrane de nylon ou de nitrocellulose une solution d'un développeur de couleur qui a un pH optimum de 6 à 9 pour une réaction de couplage avec de la naphthylamine, et à sécher ledit élément.

21. Procédé suivant la revendication 20, dans lequel ledit développeur de couleur est un sel de diazonium.

22. Procédé suivant la revendication 20 ou 21, dans lequel ledit développeur de couleur est un sel de Fast Black K ou un sel de Fast Dark Blue R.

23. Procédé suivant les revendications 20 à 22, dans lequel le premier élément est un tampon de papier cellulosique.

24. Procédé suivant l'une quelconque des revendications 20 à 23, dans lequel le premier élément contient, de plus, des moyens de fixation d'échantillon.

25. Procédé suivant la revendication 24, dans lequel les moyens de fixation d'échantillon sont des fossettes imprimées dans le premier élément.

26. Procédé suivant l'une quelconque des revendications 20 à 25, dans lequel un tampon Tris est utilisé pour confectionner la solution de BANA.

27. Procédé suivant l'une quelconque des revendications 20 à 26, dans lequel la solution du développeur de couleur contient, de plus, un agent stabilisant polymère.

28. Equipement destiné à être utilisé dans une analyse en phase solide pour la détection d'hydrolyse N-benzoyl-DL-arginine-2-naphthylamide (BANA) qui comprend:
un premier élément sur lequel une solution de N-benzoyl-DL-arginine-2-naphthylamide (BANA) tamponnée à un pH de 8 à 9 a été absorbée; et
un second élément comprenant une membrane de nylon ou de nitrocellulose sur lequel une solution d'un développeur de couleur qui a un pH optimum de 6 à 9 pour une réaction de couplage avec de la naphthylamine a été absorbée.

29. Equipement suivant la revendication 28, dans lequel ledit développeur de couleur est un sel de diazonium.

30. Equipement suivant la revendication 28 ou 29, dans lequel ledit développeur de couleur est un sel de Fast Black K ou un sel de Fast Dark Blue R.

31. Equipement suivant les revendications 28 à 30, dans lequel chaque élément est emballé de manière à interdire toute entrée de lumière, d'air, et/ou d'autres contaminants sur sa surface avant son utilisation dans la méthode revendiquée dans les revendications 9 à 17.

32. Equipement suivant les revendications 28 à 31, dans lequel le premier élément comprend une matière en papier cellulosique ou de polyester.

33. Equipement suivant les revendications 28 à 32, dans lequel le premier élément contient des moyens de fixation d'échantillon adaptés pour recevoir les échantillons de plaque sous-gingivale.

34. Equipement suivant la revendication 33, dans lequel les moyens de fixation d'échantillon sont des fossettes imprimées dans le premier élément.

35. Equipement suivant les revendications 28 à 34, dans lequel le second élément comprenant une membrane de nylon ou de nitrocellulose contient, de plus, un agent stabililisant polymère.

36. Equipement suivant les revendications 26 à 35, dans lequel chaque élément est fixé par collage à une matière robuste de renfort en plastique, etc.

37. Equipement suivant la revendication 36, dans lequel ladite matière de renfort est une carte de polystyrène.

38. Equipement suivant l'une quelconque des revendications 36 ou 37, dans lequel les éléments sont fixés symétriquement sur la matière de renfort de chaque côté d'une ligne de perforations de manière que, si la matière de renfort est repliée le long de ladite ligne de perforations, lesdits premier élément et second élément peuvent être directement mis en contact.

39. Premier élément destiné à être utilisé dans une analyse en phase solide pour la détection de l'hydrolyse de N-benzoyl-DL-arginine-2-naphthylamide (BANA) quand on le combine avec un second élément qui contient un développeur de couleur absorbée, dans lequel une solution de BANA tamponnée avec un pH de 6 à 9 est absorbée dans ledit premier élément.
